# EUROPEAN PATENT SPECIFICATION

(11) **EP 2 005 954 B1**
(45) Date of publication and mention of the grant of the patent: **24.10.2012**
(21) Application number: 07737319.9
(22) Date of filing: 22.02.2007
(51) Int. Cl.: A61K 31/196, A61K 31/198, A61K 31/20, A61K 31/337, A61K 31/513, A61K 31/53, A61K 31/573, A61K 31/704, A61K 33/36, A61P 35/00, A61P 43/00

(54) **KIT FOR CANCER THERAPY AND PHARMACEUTICAL COMPOSITION FOR CANCER THERAPY**
KIT FÜR DIE KREBSTHERAPIE UND PHARMAZEUTISCHE ZUSAMMENSETZUNG ZUR KREBSTHERAPIE
KIT POUR LA THÉRAPIE DU CANCER ET COMPOSITION PHARMACEUTIQUE POUR LA THÉRAPIE DU CANCER

(30) Priority: 23.03.2006 JP 2006080691; 13.02.2007 JP 2007032777
(43) Date of publication of application: 24.12.2008
(60) Divisional application: 11184021.1
(73) Proprietor: TMRC Co., Ltd., Tokyo 107-0052 (JP)
(72) Inventor: EKIMOTO, Hisao, Tokyo 115-0042 (JP)
(74) Representative: Jones, Helen M.M.
(86) International application number: PCT/JP2007/053326
(87) International publication number: WO 2007/108272

(56) References cited:
- EP-A- 0 396 184
- WO-A2-2004/093856
- WO-A2-2005/027842
- WO-A2-2005/039490
- JP-A- 07 215 882
- JP-A- 11 012 175
- JP-A- 2000 506 489
- JP-A- 2004 528 316
- JP-A- 2005 512 961
- DEBRUYNE F M J ET AL: "Ketoconazole high dose (H.D.) in advanced prostatic cancer : A comparison of two dosage regimens" JOURNAL OF STEROID BIOCHEMISTRY, PERGAMON PRESS PLC, GB, vol. 28, 1 January 1987 (1987-01-01), page 81, XP023420229 ISSN: 0022-4731 [retrieved on 1987-01-01]
- HO A D ET AL: "Combination of low-dose cytarabine and 13-cis retinoic acid in the treatment of myelodysplastic syndromes" LEUKEMIA RESEARCH, NEW YORK,NY, US, vol. 11, no. 11, 1 January 1987 (1987-01-01), pages 1041-1044, XP022921542 ISSN: 0145-2126 [retrieved on 1987-01-01]
- "986 PUBLICATION Clinical study of combining arsenic trioxide (As2O3), all-trans retinoic acid (ATRA) and idarubicin (IDA) for induction therapy on the patients with relapsed acute promyelocytic leukemia(APL)" EUROPEAN JOURNAL OF CANCER. SUPPLEMENT, PERGAMON, OXFORD, GB, vol. 3, no. 2, 1 October 2005 (2005-10-01), page 284, XP005133100 ISSN: 1359-6349
- GANSLMAYER MARION ET AL: "The combination of tamoxifen and 9cis retinoic acid exerts overadditive anti-tumoral efficacy in rat hepatocellular carcinoma" JOURNAL OF HEPATOLOGY, vol. 40, no. 6, June 2004 (2004-06), pages 952-956, XP009118834 ISSN: 0168-8278
- LEUNG MUN-FAI ET AL: "The combined differentiating effect of retinoic acid and vincristine on acute promyelocytic leukemia" LEUKEMIA RESEARCH, vol. 21, no. 1, 1997, pages 81-84, XP009118839 ISSN: 0145-2126
- JOZAN SUZANNE ET AL: "Cytotoxic effect of interferon-alpha2a in combination with all-trans retinoic acid or cisplatin in human ovarian carcinoma cell lines" ANTI-CANCER DRUGS, vol. 9, no. 3, March 1998 (1998-03), pages 229-238, XP009118840 ISSN: 0959-4973
- GANSLMAYER MARION ET AL: "Combined tamoxifen and retinoids affect intratumoral apoptosis rate in a rat HCC model" GASTROENTEROLOGY, vol. 126, no. 4, Suppl. 2, April 2004 (2004-04), page A597, XP009118835 & DIGESTIVE DISEASE WEEK/105TH ANNUAL MEETING OF THE AMERICAN-GASTROENTEROLOGICAL-ASSOCIATION; NEW ORLEANS, LA, USA; MAY 16 20, 2004 ISSN: 0016-5085
- CLERICI CARLO ET AL: "Treatment with all-trans retinoic acid plus tamoxifen and vitamin E in advanced hepatocellular carcinoma" ANTICANCER RESEARCH, vol. 24, no. 2C, March 2004 (2004-03), pages 1255-1260, XP009118836 ISSN: 0250-7005
- ANZANO M A ET AL: "CHEMOPREVENTION OF MAMMARY CARCINOGENESIS IN THE RAT: COMBINED USE OF RALOXIFENE AND /-CIS-RETINOIC ACID" JOURNAL OF THE NATIONAL CANCER INSTITUTE, OXFORD UNIVERSITY PRESS, GB, vol. 88, no. 2, 17 January 1996 (1996-01-17), pages 123-125, XP002054376 ISSN: 0027-8874
- EMI N.: 'Kyusei Zen Kotsudui Kyusei Hakketsubyo ni Taisuru Shinki Chiryoho' BESSATSU IGAKU NO AYUMI 15 September 2005, pages 446 - 448, XP003017982
- SAHARA N. ET AL.: 'Am-80, A Hissan ni yoru Kyusei Zen Kotsudui Kyusei Hakketsubyo no Chiryo' RINSHOI vol. 31, no. 12, 10 December 2005, pages 1920 - 1923, XP003017983
- NANRI T. ET AL.: 'Kyusei Zen Kotsuduisei Hakketsubyo (Actepromyelocytic leukemia)' HEMATOLOGY & ONCOLOGY vol. 52, no. 1, 28 January 2006, pages 10 - 19, XP003017984
- KAWAKITA T. ET AL.: 'Kyusei Zen Kotsudui Kyusei Hakketsubyo Chiryo no Saikin no Hatten (Recent advances in the treatment of acute promyclocytic leukemia)' BIO CHIMICA vol. 20, no. 13, 10 December 2005, pages 1153 - 1158, XP003017985
- YANADA M. ET AL.: '<<Hyojunteki Chiryoho to sono Mondaiten>>, Kyusei Zen Kotsuduikyusei Hakketsubyo' NAIKA vol. 92, no. 3, 01 September 2003, pages 464 - 469, XP003017986
- KAGECHIKA H.: 'Gosei Tetinoids to sonorRinsho Oyo (Synthetic tetionoids and their clinical applications)' HEMATOLOGY & ONCOLOGY vol. 51, no. 3, 28 September 2005, pages 277 - 282, XP003017987
- TALLMAN M.S. ET AL.: 'Retinoids in Cancer Treatment' J. CLIN. PHARMACOL. vol. 32, no. 10, October 1992, pages 868 - 888, XP001000254
- KUROKI T. ET AL.: 'Koganzei no Sayo Kiko' IWANAMI KOZA GENDAI IGAKU NO KISO 10 DAI 7 KAI HAIHON SAIBO ZOSHOKU TO GAN, KABUSHIKI KAISHA IWANAMI SHOTEN 29 June 1999, pages 199 - 212, XP003017988
- KALEMKERIAN G.P. ET AL.: 'Activity of Fenretinide plus chemotherapeutic agents in small-cell lung cancer cell lines' CANCER CHEMOTHER. PHARMACOL. vol. 43, no. 2, February 1999, pages 145 - 150, XP002950455
- FORMELLI F. ET AL.: 'Synthetic retinoid Fenretinide is effective against a human ovarian carcinoma xenograft and potentiates Cisplatin activity' CANCER RESEARCH vol. 53, no. 22, 15 November 1993, pages 5374 - 5376, XP008130036
- SHUDO K. ET AL.: 'kan Shuyo Chiroyozai toshite no Gosei Retinoid Kaihatsu-TAK-101 no Kiso to Rinsho eno Tenbo (Synthetic retinoid development as hepatic tumor therapeutic agent. Basis of TAC-101 and prospect of its clinical application)' THE JOURNAL OF CLINICAL SCIENCE vol. 33, no. 6, June 1997, pages 721 - 727, XP003017990

## Description

### Technical Field

The present invention relates to a kit for inhibiting the growth of tumors and cancers in mammals, and more specifically relates to a kit for cancer treatment and a pharmaceutical composition for cancer treatment for inhibiting the growth of cancers and tumors in mammals, in particular, human and warm-blooded animals.

### Background Art

Cancers are the leading cause of death in animals and human. Many types of chemotherapeutic agents have been shown to be effective against cancer and tumor cells, but unfortunately, many of these agents have a problem that they also destroy normal cells. Despite improvements in the field of cancer treatment, the leading therapies to date are surgery, radiation, and chemotherapy.

Chemotherapeutic approaches are recognized to be effective against cancers that are metastatic or are particularly progressive. The exact mechanism for the action of these chemotherapeutic agents are not always known. Moreover, while some chemotherapeutic agents significantly reduce tumor masses after treatment with such agents, it happens often that they unfortunately cannot be administered again to the same patients when the tumors recurred. In addition, since the width between a therapeutically effective dose and a maximum tolerated dose of chemotherapeutic agents is generally narrow, the administration amount must be determined meticulously.

Today, retinoids are known to be involved in signal transduction systems of immune cells and cause various immune reactions. Recently, it has been reported that retinoids suppress Th1 expression on T cells and enhance Th2 expression (Non-Patent Document 1).

Furthermore, it has been recently reported that a combination use of an antiangiogenic drug and a chemotherapeutic agent shows a high effectiveness (Non-Patent Document 2).
Non-Patent Document 1: M. Iwata, et al., Retinoic acids exert direct effects on T cells to suppress Th1 development and enhance Th2 development via retinoic acid receptors. International Immunology, 15(8): pp.1017-1025, 2003.
Non-Patent Document 2: Sengupta, S., et al., Temporal atrgeting of tumor cells and neovasculature with a nanoscale delivery system. Nature, 436: pp.568-572, 2005.

A brief description of the relevant prior art is given below.

J. Ma et al provided a clinical study of combining arsenic trioxide, all-*trans* retinoic acid and idarubicin for induction therapy on patients with relapsed acute promyelocytic leukemia (European Journal of Cancer Supplement, Hergomen, Oxford GB, volume 3, no. 2, 1 October 2005, page 284.)

Ganslmayer, Marion et al., showed the effects of 9-cis retinoic acid and tamoxifen for treatment of hepatocellular cells which was shown to be effective only in high concentration. (Journal of Hepatology, volume 40, no.6, June 2004, pages 952-956.)

Leung, Mun-fai et al., showed that HL60 cells exposed to all-*trans* retinoic acid after treatment with a non-cytotoxic concentration of vincristine resulted in granulocytic maturation and differentiation which suggested that there might be synergistic action with all-*trans* retinoic acid in the treatment of acute promyelocytic leukemia. (Leukemia Research, volume 21, no. 1, 1997, pages 81-84.)

Patent document US6624154 discloses compositions comprising a retinoid X receptor agonist, for instance the synthetic retinoid represented by formula (I), and an agent capable of activating protein kinase A. The invention also relates to methods of treating hyperproliferative diseases by administering the composition.

Patent document W09846076 discloses a method of inhibiting endothelin-1 in a subject comprising administering to the subject an inhibiting amount of a suitable retinoid or retinoid related molecule, for instance the synthetic retinoid represented by formula (I), and a method of treating pain and diseases associated with the presence of increased levels of endothelin-1.

### Disclosure of Invention

### Problems to be Solved by the Invention

As described above, since that retinoids cause various immune reactions and a combination use of an agent targeting tumor vessels and a chemotherapeutic agent shows a high effectiveness on tumor cells as a target, it is expected to reduce toxicity against normal cells in order to enhance specificity to tumor cells. However, at the same time, an administration method that does not act on normal cells but enhances cytotoxicity against tumor cells more than ever is highly expected.

Accordingly, it is an object of the present invention to provide a kit for cancer treatment and a pharmaceutical composition for cancer treatment for inhibiting the growth of tumors and cancers in mammals more than ever.

### Means for Solving the Problems

The present inventor has conducted intensive studies for solving the above-mentioned problems and has found that a synthetic retinoid is particularly useful for suppression of the growth of cancers and tumors and further found that a high effectiveness can be obtained by formulating the synthetic retinoid to a kit preparation that is sequentially used in a combination with another chemotherapeutic agent that is effective for reducing tumor size (debulking), and side effects of the kit preparation are milder compared to those in a combination use of only chemotherapeutic agents. The present invention has been thus completed.

That is, the present invention relates to the following (1) to (8):
1. A kit for use in cancer treatment comprising a combination of two different drugs in a kit formulation, wherein a first drug contains a synthetic retinoid represented by the following formula (I): 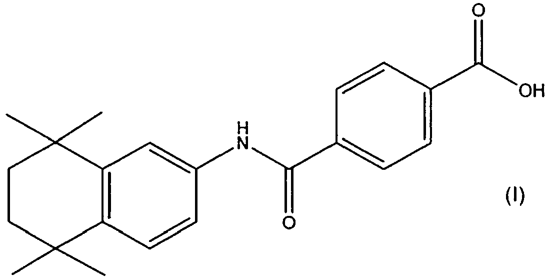 or a pharmaceutically acceptable organic or inorganic acid addition salt thereof, and a second drug contains a chemotherapeutic agent for cancer treatment selected from a group consisting of prednisolone, arsenous acid, 5-aza-2'-deoxycytidine, melphalan, dexamethasone, and valproic acid.
2. The kit for use in cancer treatment according to claim 1, wherein the first drug contains the synthetic retinoid or a pharmaceutically acceptable organic or inorganic acid addition salt thereof at 0.5 to 30 mg per human or animal, and the second drug contains the chemotherapeutic agent at 1.0 to 1000 mg per human or animal.
3. The kit for use in cancer treatment according to claim 1 or 2, wherein the first drug contains the synthetic retinoid or a pharmaceutically acceptable organic or inorganic acid addition salt thereof at a dosage for 21 days or 28 days, and the second drug contains the chemotherapeutic agent at a dosage for treatment for 1 to 21 days or 28 days.
4. The combination of a first drug containing a synthetic retinoid represented by the following formula (I): 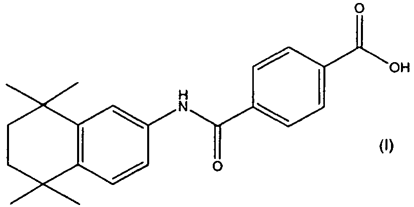 or a pharmaceutically acceptable organic or inorganic acid addition salt thereof, and a second drug containing a chemotherapeutic agent for cancer treatment selected from a groups consisting of prednisolone, arsenous acid, 5-aza-2'-deoxycytidine, melphalan, dexamethasone, and valproic acid, for use in a method of treatment of cancer tumor in which the first drug is administered and simultaneously or subsequently the second drug is administered whereby the tumor is reduced in size.
5. The combination according to claim4, wherein the second drug is administered after the administration of the first drug and observation of a reduction in tumor size.
6. The combination according to claims 4 or 5, wherein the first drug contains the synthetic retinoid or pharmaceutically acceptable organic or inorganic acid addition salt thereof at 0.5 to 30 mg per human or animal, and the second drug contains a chemotherapeutic agent at 1.0 to 1000 mg per human or animal.
7. The combination according to claims 4 to 6, wherein the first drug contains a synthetic retinoid or a pharmaceutically acceptable organic or inorganic acid addition salt thereof at a dosage for 21 days or 28 days, and the second drug contains a chemotherapeutic agent at a dosage for treatment for 1 to 21 days or 28 days.
8. The combination according to claim 4, wherein the first drug and the second drug are simultaneously administered.

A synthetic retinoid is first administered and then a chemotherapeutic agent is administered, and thereby the chemotherapeutic agent showing cytotoxicity can reduce or contract the size of a tumor mass more than ever. In addition, the method of simultaneously administering a synthetic retinoid and a chemotherapeutic agent is useful. Examples of cancers that are treated with the present invention include blood cancers such as acute myelocytic leukemia, acute lymphocytic leukemia, multiple myeloma, and non-Hodgkin's lymphoma; lung cancer; digestive cancers such as colon cancer, gastric cancer, liver cancer, pancreatic cancer, and bile duct cancer; breast cancer; prostate cancer; ovary cancer; and uterine cancer.

### Brief Description of Drawings

Fig. 1 is a graph showing a synergistic effect on growth-suppressing activity in a combination use of TM-411 and As₂O₃ for human acute promyelocytic leukemia cell line HL-60.
Fig. 2 is a graph showing a synergistic effect on growth-suppressing activity in a combination use of TM-411 and 5-AZ for human acute promyelocytic leukemia cell line HL-60.
Fig. 3 is a graph showing an additive effect and a synergistic effect on growth-suppressing activity in a combination use of TM-411 and Mel for human multiple myeloma cell line RPMI8226.
Fig. 4 is a graph showing a synergistic effect on growth-suppressing activity in a combination use of TM-411 and PSL for human multiple myeloma cell line RPMI8226.
Fig. 5 is a graph showing a synergistic effect on growth-suppressing activity in a combination use of TM-411 and DEX for human multiple myeloma cell line RPMI8226.
Fig. 6 is a graph showing a synergistic effect on growth-suppressing activity in a combination use of TM-411 and 5-AZ for human multiple myeloma cell line RPMI8226.
Fig. 7 is a graph showing an additive effect and a synergistic effect on growth-suppressing activity in a combination use of TM-411 and VPA for human hepatocellular carcinoma cell line JHH-7.

### Best Modes for Carrying Out the Invention

In the kit of the present invention, examples of the synthetic retinoid or a pharmaceutically acceptable organic or inorganic acid addition salt thereof contained in the first drug are preferably benzoic acid derivatives or pharmaceutically acceptable organic or inorganic acid addition salts thereof and particularly preferably a benzoic acid derivative represented by the following formula (I): 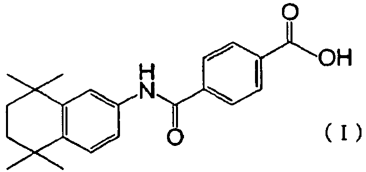 or a pharmaceutically acceptable organic or inorganic acid addition salt thereof.

The synthetic retinoid is an agonist or an antagonist of a retinoic acid receptor (RAR α, β, or γ) or a retinoid receptor (RXR α, β, or γ), or a pharmaceutically acceptable organic or inorganic acid addition salt thereof.

In addition, in the kit of the present invention, examples of the DNA interactive agent as the chemotherapeutic agent contained in the second drug include alkylation agents such as , melphalan, and is preferably orally available DNA interactive agents: melphalan.

Some of the antimetabolites as the chemotherapeutic agents inhibit DNA replication by inhibiting biosynthesis of deoxyribonucleoside triphosphate, which is the immediate precursor of DNA synthesis. In addition, examples of DNA methylation inhibitors include 5-aza-2'-deoxycytidine (hereinafter, abbreviated to "5-AZ").

Examples of the anticancer antibiotic as the chemotherapeutic agent include valproic acid used as an antiepileptic agent and arsenous acid, which inhibits membrane permeability of mitochondria, can be also used as the anticancer antibiotic as the chemotherapeutic agents.

Examples of the enzyme inhibitor as the chemotherapeutic agent include histone deacetylase inhibitors such as valproic acid and DNA methylation inhibitors such as 5-AZ, and are preferably orally available valproic acid, and 5-AZ.

Examples of the steroids used in hormone therapy include dexamethasone, and examples of the hormone or anti-hormone antagonist include Prednisolone.

The first drug and the second drug in the kit of the present invention are useful for inhibiting the growth of cancers and other tumors in human or animals by administering effective doses of them orally, rectally, topically, or parenterally, or by injection to a vein or a tumor. Each drug of such a unit may be a solid such as pills, tablets, capsules, or liposome; gel; or a liquid that is suitable for oral, rectal, topical, intravascular, or parental administration, or for injection to the inside or near tumor cells (refer to Cancer Chemotherapy Handbook, 2nd edition, pp. 15-34, Appleton & Lange (Connecticut, 1994)).

The kit of the present invention preferably includes the first drug containing a synthetic retinoid or a pharmaceutically acceptable organic or inorganic acid addition salt thereof at a dosage for 21 days or 28 days and the second drug containing a chemotherapeutic agent at a dosage for treatment for 1 to 21 days or 28 days.

Specifically, when such a kit is used for therapy, effective amounts can be appropriately determined according to the therapy of a specific cancer or tumor type to be treated, and the route of applying the effective amount also varies depending on the tumor to be treated. Preferably, a synthetic retinoid is administered first to reduce the size of a cancer or tumor mass. In general, it takes from two to eight weeks. The reduction of the tumor or cancer cells in size is less than 50% of the original. Then, when the reduction of the tumor in size is observed, a chemotherapeutic agent is administered. Since the synthetic retinoid is relatively safe, it can be administered for from two weeks to one year, if necessary, for maintaining the effectiveness for suppressing cancer regrowth.

### Examples

The present invention will now be described with reference to Examples.

### Example 1

### (Effects of TM-411, prednisolone, and a combination use of TM-411 and prednisolone on human multiple myeloma U266 in NOG mice)

Prednisolone (hereinafter, abbreviated to "PSL"), which is a general name of that manufactured by Shionogi & Co., Ltd., was used as a chemotherapeutic agent.

Seven-week-old NOD/SCID/γc^{null} (NOG) mice were divided into five groups of ten mice each (nine mice only in a control group), and 2 × 16⁶ cells of multiple myeloma cell line U266 were transplanted to each mouse via tail vein injection (the 0th day). The first group was used as a non-treated control group; in the second group, 1 mg/kg of TM-411 was orally administered for 28 days from the first day; in the third group, 3 mg/kg of TM-411 was orally administered for 28 days from the first day; in the fourth group, 7.5 mg/kg of PSL was orally administered for 8 days from the 21st day; and in the fifth group, 1 mg/kg of TM-411 was orally administered for 28 days from the first day and 7.5 mg/kg of PSL was orally administered in addition to the TM-411 for 8 days from the 21st day. Blood concentration of human IgE was measured 6 weeks after the transplantation of U266. Table 4 shows the results.

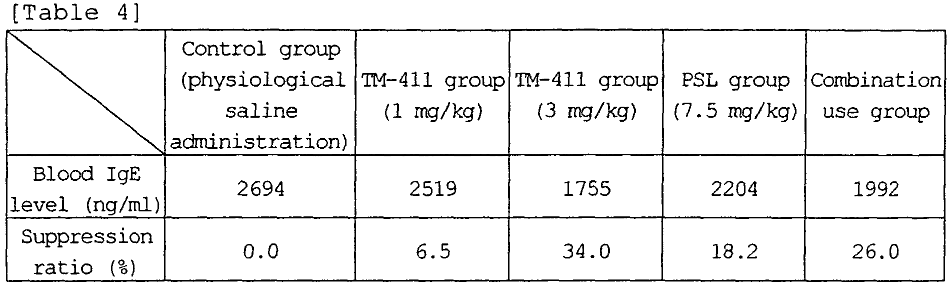

As shown in Table 4, both TM-411 and PSL decreased blood concentration of human IgE by administering TM-411 or PSL to the NOG mice transplanted with human multiple myeloma U266. The combination use of TM-411 and PSL showed a synergistic effect on the decrease of the blood concentration of human IgE.

### Example 2

### (Effect of a combination use of TM-411 and arsenous acid on human acute promyelocytic leukemia cell line HL-60)

Arsenous acid (hereinafter, abbreviated to "As₂O₃") manufactured by Sigma-aldrich Japan K.K. was used as a chemotherapeutic agent.

Human acute promyelocytic leukemia cell line HL-60 cells were cultured in Iscove's modified Dulbecco's medium (GIBCO Laboratories) containing 20% bovine fetal serum, and then TM-411 and As₂O₃ were added thereto. The synergistic effect on the growth-suppressing activity was verified by MTT (5% thiazolyl blue tetrazolium bromide) assay.

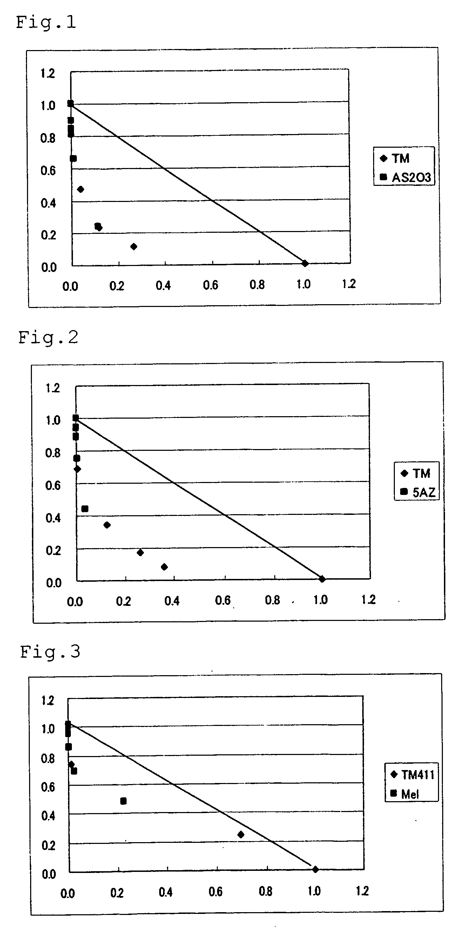

In Fig. 1, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of As₂O₃ is assumed to be 1. Points on the straight line connecting the points of 1.0 on both axes mean an additive effect, and points on the underside of the line mean a synergistic effect. As shown in Fig. 1, it was observed that the combination use of TM-411 and As₂O₃ had a synergistic effect on the growth-suppressing activity in human acute promyelocytic leukemia cell line HL-60.

### Example 3

### (Effect of a combination use of TM-411 and 5-AZ, a DNA methylation inhibitor, on human acute promyelocytic leukemia cell line HL-60)

5-AZ manufactured by CALBIOCHEM was used as a DNA methylation inhibitor.

The synergistic effect on the growth-suppressing activity in the addition of TM-411 and 5-AZ was investigated by the same technique as that in Example 5.

In Fig. 2, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of 5-AZ is assumed to be 1. Points on the straight line connecting the points of 1.0 on both axes mean an additive effect, and points on the underside of the line mean a synergistic effect. As shown in Fig. 2, it was observed that the combination use of TM-411 and 5-AZ had a synergistic effect on the growth-suppressing activity in human acute promyelocytic leukemia cell line HL-60.

### Example 4.

### (Effect of a combination use of TM-411 and melphalan on human multiple myeloma cell line RPMI8226)

Melphalan (hereinafter, abbreviated to "Mel") was used as a chemotherapeutic agent.

Human multiple myeloma cell line RPMI8226 cells were cultured in RPMI1640 medium containing 10% bovine fetal serum, and then TM-411 and Mel were added thereto. The synergistic effect in the growth-suppressing activity was verified.

In Fig. 3, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of Mel is assumed to be 1. Points on the straight line connecting the points of 1.0 on both axes mean an additive effect, and points on the underside of the line mean a synergistic effect. As shown in Fig. 3, it was observed that the combination use of TM-411 and Mel had an additive to synergistic effect on the growth-suppressing activity in human multiple myeloma cell line RPMI8226.

### Example 5

### (Effect of a combination use of TM-411 and PSL on human multiple myeloma cell line RPMI8226)

PSL was used as a chemotherapeutic agent.

The synergistic effect on the growth-suppressing activity in the addition of TM-411 and PSL was verified by the same technique as that in Example 7.

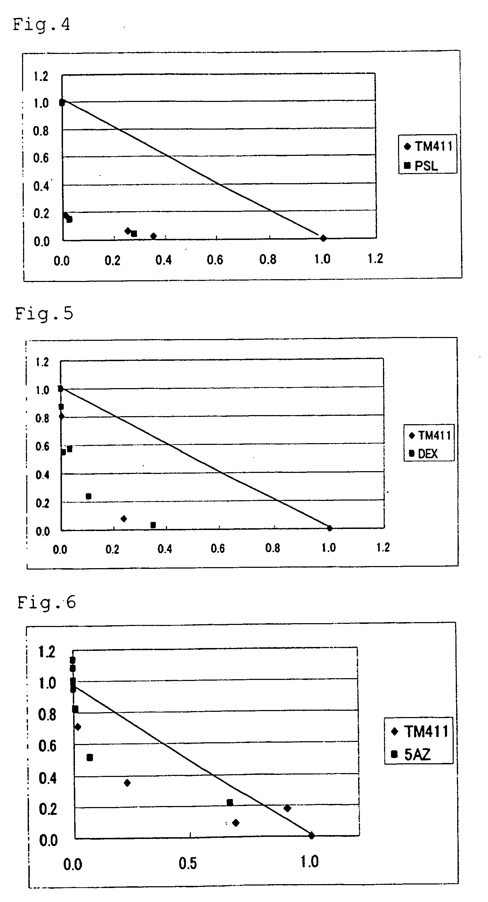

In Fig. 4, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of PSL is assumed to be 1. Points on the straight line connecting the points of 1.0 on both axes mean an additive effect, and points on the underside of the line mean a synergistic effect. As shown in Fig. 4, it was observed that the combination use of TM-411 and PSL had a synergistic effect on the growth-suppressing activity in human multiple myeloma cell line RPMI8226.

### Example 6

### (Effect of a combination use of TM-411 and dexamethasone on human multiple myeloma cell line RPMI8226)

Dexamethasone (hereinafter, abbreviated to "DEX") was used as a chemotherapeutic agent.

The synergistic effect on the growth-suppressing activity in the addition of TM-411 and DEX was verified by the same technique as that in Example 7.

In Fig. 5, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of DEX is assumed to be 1. Points on the straight line connecting the points of 1.0 on both axes mean an additive effect, and points on the underside of the line mean a synergistic effect. As shown in Fig. 5, it was observed that the combination use of TM-411 and DEX had a synergistic effect on the growth-suppressing activity in human multiple myeloma cell line RPMI8226.

### Example 7

### (Effect of a combination use of TM-411 and 5-AZ, a methylation inhibitor, on human multiple myeloma cell line RPMI8226)

5-AZ was used as a methylation inhibitor.

The synergistic effect on the growth-suppressing activity in the addition of TM-411 and 5-AZ was verified by the same technique as that in Example 7.

In Fig. 6, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of 5-AZ is assumed to be 1. Points on the straight line connecting the points of 1.0 on both axes mean an additive effect, and points on the underside of the line mean a synergistic effect. As shown in Fig. 6, it was observed that the combination use of TM-411 and 5-AZ had a synergistic effect on the growth-suppressing activity in human multiple myeloma cell line RPMI8226.

### Example 8

### (Effect of a combination use of TM-411 and valproic acid on human hepatocellular carcinoma cell line JHH-7)

Valproic acid (hereinafter, abbreviated to "VPA") was used as a chemotherapeutic agent.

The additive and synergistic effect on the growth-suppressing activity in the addition of TM-411 and VPA was verified by the same technique as that in Example 7.

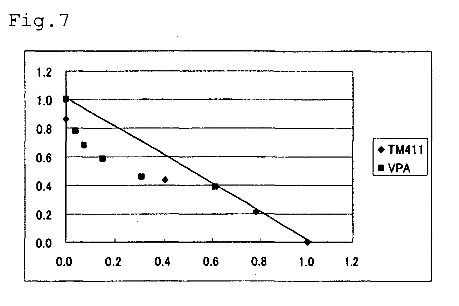

In Fig. 7, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of VPA is assumed to be 1. Points on the straight line connecting the points of 1.0 on both axes mean an additive effect, and points on the underside of the line mean a synergistic effect. As shown in Fig. 7, it was observed that the combination use of TM-411 and VPA had an additive to synergistic effect on the growth-suppressing activity in human hepatocellular carcinoma cell line JHH-7.

## Claims

1. A kit for use in cancer treatment comprising a combination of two different drugs in a kit formulation, wherein a first drug contains a synthetic retinoid represented by the following formula (I): 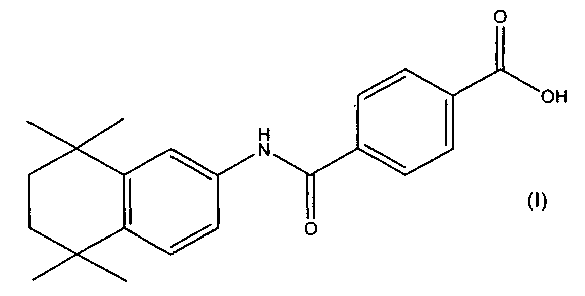 or a pharmaceutically acceptable organic or inorganic acid addition salt thereof, and a second drug contains a chemotherapeutic agent for cancer treatment selected from a group consisting of prednisolone, arsenous acid, 5-aza-2'-deoxycytidine, melphalan, dexamethasone, and valproic acid.

2. The kit for use in cancer treatment according to claim 1, wherein the first drug contains the synthetic retinoid or a pharmaceutically acceptable organic or inorganic acid addition salt thereof at 0.5 to 30 mg per human or animal, and the second drug contains the chemotherapeutic agent at 1.0 to 1000 mg per human or animal.

3. The kit for use in cancer treatment according to claim 1 or 2, wherein the first drug contains the synthetic retinoid or a pharmaceutically acceptable organic or inorganic acid addition salt thereof at a dosage for 21 days or 28 days, and the second drug contains the chemotherapeutic agent at a dosage for treatment for 1 to 21 days or 28 days.

4. The combination of a first drug containing a synthetic retinoid represented by the following formula (I): 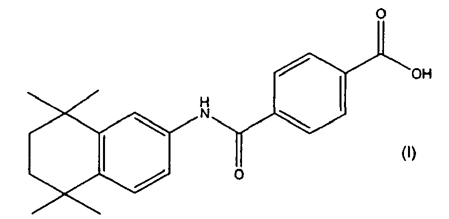 or a pharmaceutically acceptable organic or inorganic acid addition salt thereof and a second drug containing a chemotherapeutic agent for cancer treatment selected from a groups consisting of prednisolone, arsenous acid, 5-aza-2'-deoxycytidine, melphalan, dexamethasone, and valproic acid, for use in a method of treatment of cancer tumor in which the first drug is administered and simultaneously or subsequently the second drug is administered whereby the tumor is reduced in size.

5. The combination according to claim4, wherein the second drug is administered after the administration of the first drug and observation of a reduction in tumor size.

6. The combination according to claims 4 or 5, wherein the first drug contains the synthetic retinoid or pharmaceutically acceptable organic or inorganic acid addition salt thereof at 0.5 to 30 mg per human or animal, and the second drug contains a chemotherapeutic agent at 1.0 to 1000 mg per human or animal.

7. The combination according to claims 4 to 6, wherein the first drug contains a synthetic retinoid or a pharmaceutically acceptable organic or inorganic acid addition salt thereof at a dosage for 21 days or 28 days, and the second drug contains a chemotherapeutic agent at a dosage for treatment for 1 to 21 days or 28 days.

8. The combination according to claim4, wherein the first drug and the second drug are simultaneously administered.

## Patentansprüche

1. Kit zur Verwendung in der Krebsbehandlung, umfassend eine Kombination aus zwei verschiedenen Arzneimitteln in einer Kit-Formulierung, wobei ein erstes Arzneimittel ein synthetisches Retinoid enthält, das durch die folgende Formel (I) 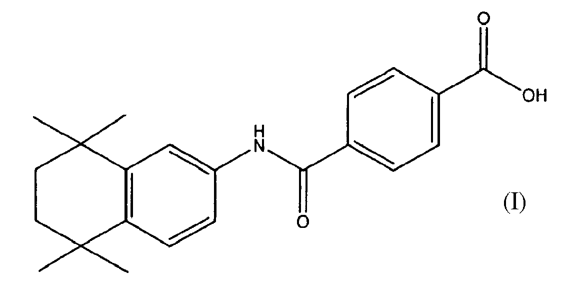 dargestellt ist, oder ein pharmazeutisch verträgliches organisches oder anorganisches Säureadditionssalz davon, und ein zweites Arzneimittel ein Chemotherapeutikum zur Krebsbehandlung enthält, das aus der Gruppe ausgewählt ist, bestehend aus Prednisolon, Arsen(II)-säure, 5-Aza-2'-desoxycytidin, Melphalan, Dexamethason und Valproinsäure.

2. Kit zur Verwendung in der Krebsbehandlung nach Anspruch 1, wobei das erste Arzneimittel das synthetische Retinoid oder ein pharmazeutisch verträgliches organisches oder anorganisches Säureadditionssalz davon zu 0,5 bis 30 mg pro Mensch oder Tier enthält, und das zweite Arzneimittel das Chemotherapeutikum zu 1,0 bis 1000 mg pro Mensch oder Tier enthält.

3. Kit zur Verwendung in der Krebsbehandlung nach Anspruch 1 oder 2, wobei das erste Arzneimittel das synthetische Retinoid oder ein pharmazeutisch verträgliches organisches oder anorganisches Säureadditionssalz davon in einer Dosierung über 21 Tage oder 28 Tage enthält und das zweite Arzneimittel das Chemotherapeutikum in einer Dosierung zur 1- bis 21-tägigen oder 28-tägigen Behandlung enthält.

4. Kombination eines ersten Arzneimittels mit einem synthetischen Retinoid, das durch die folgende Formel (I) 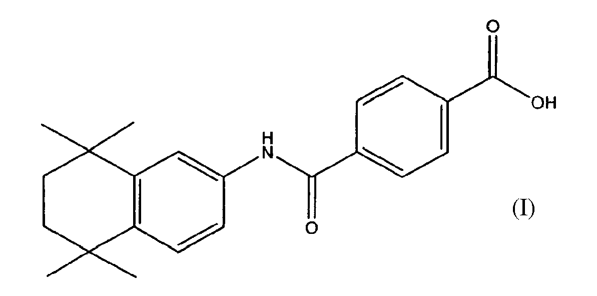 dargestellt ist, oder ein pharmazeutisch verträgliches organisches oder anorganisches Säureadditionssalz davon, und ein zweites Arzneimittel mit einem Chemotherapeutikum zur Krebsbehandlung, das aus einer Gruppe ausgewählt ist, bestehend aus Prednisolon, Arsen(III)-säure, 5-Aza-2'-desoxycytidin, Melphalan, Dexamethason und Valproinsäure, zur Verwendung in einem Verfahren zur Behandlung eines Krebstumors, wobei das erste Arzneimittel verabreicht wird und gleichzeitig oder anschließend daran das zweite Arzneimittel verabreicht wird, wodurch die Größe des Tumors reduziert wird.

5. Kombination nach Anspruch 4, wobei das zweite Arzneimittel nach der Verabreichung des ersten Arzneimittels verabreicht wird und Beobachtung einer Reduktion der Tumorgröße.

6. Kombination nach Anspruch 4 oder 5, wobei das erste Arzneimittel das synthetische Retinoid oder ein pharmazeutisch verträgliches organisches oder anorganisches Säureadditionssalz davon zu 0,5 bis 30 mg pro Mensch oder Tier enthält und das zweite Arzneimittel ein Chemotherapeutikum zu 1,0 bis 1000 mg pro Mensch oder Tier enthält.

7. Kombination nach den Ansprüchen 4 bis 6, wobei das erste Arzneimittel ein synthetisches Retinoid oder ein pharmazeutisch verträgliches organisches oder anorganisches Säureadditionssalz davon in einer Dosierung über 21 Tage oder 28 Tage enthält und das zweite Arzneimittel ein Chemotherapeutikum in einer Dosierung zur 1-bis 21-tägigen oder 28-tägigen Behandlung enthält.

8. Kombination nach Anspruch 4, wobei das erste Arzneimittel und das zweite Arzneimittel gleichzeitig verabreicht werden.

## Revendications

1. Trousse destinée au traitement du cancer comprenant une association de deux médicaments différents dans une formulation de la trousse, dans lequel un premier médicament contient un rétinoïde synthétique représenté par la formule (I) suivante : 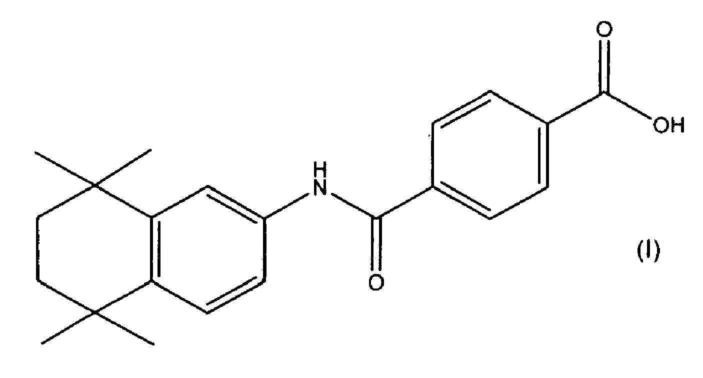 ou un sel d'addition d'acide organique ou inorganique pharmaceutiquement acceptable de celui-ci et un deuxième médicament contient un agent chimiothérapeutique pour le traitement du cancer sélectionné parmi un groupe constitué de prednisole, acide arsénieux, 5-aza-2'-déoxycytidine, melphalan, dexaméthasone et acide valproïque.

2. Trousse destinée au traitement du cancer selon la revendication 1, dans laquelle le premier médicament contient le rétinoïde synthétique ou un sel d'addition d'acide organique ou inorganique pharmaceutiquement acceptable de celui-ci à une posologie de 0,5 à 30 mg par être humain ou animal, et le deuxième médicament contient l'agent chimiothérapeutique à une posologie de 1,0 à 1000 mg par être humain ou animal.

3. Trousse destinée au traitement du cancer selon la revendication 1 ou 2, dans laquelle le premier médicament contient le rétinoïde synthétique ou un sel d'addition d'acide organique ou inorganique pharmaceutiquement acceptable de celui-ci à une posologie à administrer pendant 21 jours ou 28 jours, et le deuxième médicament contient l'agent chimiothérapeutique à une posologie à administrer pour un traitement pendant 1 à 21 jours ou 28 jours.

4. Association d'un premier médicament contenant un rétinoïde synthétique représenté par la formule (I) : 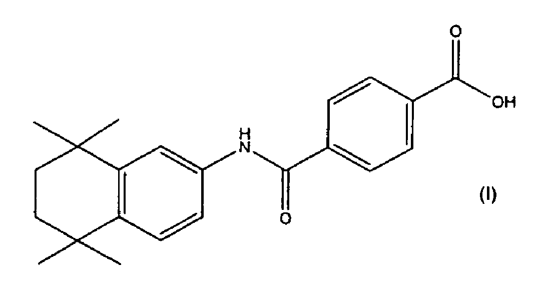 ou un sel d'addition d'acide organique ou inorganique pharmaceutiquement acceptable de celui-ci,
et un deuxième médicament contenant un agent chimiothérapeutique destiné au traitement du cancer sélectionné parmi un groupe constitué de prednisole, acide arsénieux, 5-aza-2'-déoxycytidine, melphalan, dexaméthasone et acide valproïque prévu pour une utilisation dans un procédé de traitement d'une tumeur cancéreuse dans lequel le premier traitement est administré et, simultanément ou ultérieurement, le deuxième médicament est administré si bien que la taille de la tumeur est réduite.

5. Association selon la revendication 4, dans laquelle le deuxième médicament est administré après l'administration du premier médicament et l'observation d'une réduction de la taille de la tumeur.

6. Association selon les revendications 4 ou 5, dans laquelle le premier médicament contient le rétinoïde synthétique ou un sel d'addition d'acide organique ou inorganique pharmaceutiquement acceptable de celui-ci à une posologie de 0,5 à 30 mg par être humain ou animal et le deuxième médicament contient un agent chimiothérapeutique à une posologie de 1,0 à 1000 mg par être humain ou animal.

7. Association selon les revendications 4 à 6, dans laquelle le premier médicament contient un rétinoïde synthétique ou un sel d'addition d'acide organique ou inorganique pharmaceutiquement acceptable de celui-ci à une posologie à administrer pendant 21 jours ou 28 jours et le deuxième médicament contient un agent chimiothérapeutique à une posologie à administrer pour un traitement pendant 1 à 21 jours ou 28 jours.

8. Association selon la revendication 4, dans laquelle le premier médicament et le deuxième médicament sont administrés simultanément.
